(19) Europäisches Patentamt
European Patent Office
Office européen des brevets

(11) Veröffentlichungsnummer: **0 370 448 B1**

(12) # EUROPÄISCHE PATENTSCHRIFT

(45) Veröffentlichungstag der Patentschrift: **09.03.94**

(51) Int. Cl.[5]: **C07D 235/26**

(21) Anmeldenummer: **89121485.0**

(22) Anmeldetag: **21.11.89**

(54) **Verfahren zur Herstellung von Benzimidazolonen.**

(30) Priorität: **25.11.88 DE 3839743**

(43) Veröffentlichungstag der Anmeldung:
**30.05.90 Patentblatt 90/22**

(45) Bekanntmachung des Hinweises auf die Patenterteilung:
**09.03.94 Patentblatt 94/10**

(84) Benannte Vertragsstaaten:
**BE CH DE ES FR GB IT LI NL**

(56) Entgegenhaltungen:
**DD-A- 155 616**
**DE-A- 2 819 458**

**CHEMICAL ABSTRACTS, Band 106, Nr. 23, 8. Juni 1987, Columbus, Ohio, USABOULLAIS,C. et al. "Synthesis of 4-(3-t-butylamino-2-hydro-xypropoxy)benzimidazol- 2(11C)-one (CGP 12177)" Seite 736, Spalte 1,Zusammenfassung Nr. 196 324h**

**CHEMICAL ABSTRACTS, Band 67, Nr. 25, 18. Dezember 1967, Columbus, Ohio, USAINUKAI K. et al. "Reaction of diaminobenzotrifluorides with phosgene." Seite10981, Spalte 2, Zusammenfassung Nr. 116 664g**

(73) Patentinhaber: **HOECHST AKTIENGESELLSCHAFT**

**D-65926 Frankfurt(DE)**

(72) Erfinder: **Meier, Michael, Dr.**
**Franz-Rücker-Allee 13**
**D-6000 Frankfurt am Main 90(DE)**
Erfinder: **Semler, Günther, Dr.**
**Johann-Strauss-Strasse 44**
**D-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur Herstellung von Benzimidazolonen durch Umsetzung von gegebenenfalls substituierten o-Phenylendiaminen mit Phosgen in Wasser, indem man die Umsetzung bei einem konstanten pH-Wert Zwischen 6 bis 12 durchführt.

Es ist bekannt, daß Benzimidazolon in guten Ausbeuten aus o-Phenylendiamin mit Harnstoff hergestellt verden kann (EP 13859). Ein Nachteil dieses Verfahrens ist der Zwangsanfall großer Mengen Ammoniak.

In DE 3 124 618 wird ein Verfahren beschrieben, bei dem substituierte o-Nitroaniline mit Kohlenmonoxid unter Druck und Zusatz von Selen und Base in ca. 90 % Ausbeute die entsprechenden Benzimidazolone ergeben. Auch bei diesem Verfahren entstehen durch das verwendete Selen und die Verwendung von Lösungsmitteln sowie organischen Basen erhebliche ökologische Probleme. Unter dem gleichen Aspekt ist die Umsetzung von o-Phenylendiamin mit Kohlenmonoxid unter Verwendung von Schwefel, Triethylamin und Tetrahydrofuran zu sehen (J 57/134470). Ein weiterer Nachteil dieser genannten Kohlenmonoxidverfahren sind hohe Reaktionsdrücke, wodurch ein hoher apparativer Aufwand erforderlich wird. Die Umsetzung von o-Phenylendiamin mit Diethylcarbonat führt in lediglich 15 %igem Umsatz zu Benzimidazolon und ist deshalb für die technische Nutzung nicht geeignet (DE 2 528 368, Beispiel 5).

Die Umsetzung von substituiertem o-Phenylendiamin mit Phosgen in Wasser führt ebenfalls nur in Ausbeuten von 18-62 % zu den entsprechenden Benzimidazolonen (DE 2 905 876, Beispiel 1; EP 784, Beispiel 1). Auch in wäßriger Salzsäure ergibt die Umsetzung von o-Phenylendiaminen mit Phosgen nur in 60 %iger Ausbeute die reinen Benzimidazolone (US 2 933 503, DE 2 705 892). P. Bouchet et al. (J. Heterocycl. Chem. 15, 625 (1978)) erhielten bei dieser Arbeitsweise maximal 80 % Ausbeute. Wird das substituierte o-Phenylendiamin in Natronlauge (ca. 7facher Überschuß) mit Phosgen umgesetzt, so erhält man das entsprechende Benzimidazolon in 80 %iger Ausbeute. Außerdem kommt es bei dem ständig hohen pH-Wert zu erheblicher Hydrolyse von Phosgen und damit zu einem zusätzlichen Salzanfall (S.M. Gadekar, J.C. Frederik, J. Org. Chem. 27, 1383, (1962)). In organischen Lösungsmitteln (siehe z.B. L.C. March et al., J. Heterocycl. Chem. 7, 39 (1970), W.J. Schnabel et al., J. Org. Chem. 34, 1162 (1969)) sind die Ausbeuten mit höchtens 60 % ebenfalls schlecht.

Zusammenfassend läßt sich sagen, daß bei den bekannten Phosgen-Verfahren zur Herstellung von Benzimidazolonen die Ausbeuten generell niedrig sind. Außerdem ist meist ein deutlicher Phosgen-Überschuß notwendig.

Demgegenüber wurde nun gefunden, daß man Benzimidazolone der allgemeinen Formel (1)

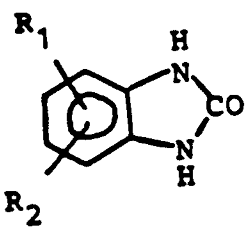

(1)

in welcher R und R gleich oder verschieden sind und 1 2 Wasserstoff- oder Halogenatome, wie beispielsweise Fluor-, Chlor- oder Bromatome, Alkyl(C -C )-, Alkoxy($C_1$-$C_4$)-, 1 4 Trifluormethyl-, Phenyl-, Phenoxy- oder Nitrogruppen bedeuten, in guten Ausbeuten und hohen Reinheiten herstellen kann, indem man die Umsetzung von o-Phenylendiaminen der allgemeinen Formel (2)

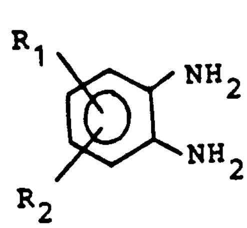

(2)

in welcher $R_1$ und $R_2$ die vorstehend genannten Bedeutungen haben, mit Phosgen in Wasser in Gegenwart einer Alkali- oder Erdalkalibase oder eines Salzes aus einem Alkali- oder Erdalkalihydroxid und einer schwachen anorganischen oder organischen Säure oder einer Mischung solcher Salze oder eines geeigneten Puffersystems bei einem konstanten pH-Wert Zwischen 6 bis 12, vorzugsweise von 6,5 bis 11,

besonders bevorzugt von 8 bis 10, und einer Temperatur von 20 bis 100 °C durchführt.

Bei pH-Werten kleiner als 6,5 kommt es zum Ausgasen und damit zu einem zusätzlichen Verbrauch von Phosgen. Außerdem verschlechtern sich bei niedrigeren pH-Werten Ausbeute und Qualität der Benzimidazolone. Bei pH-Werten über 10,5 werden durch die unerwünschte Hydrolyse von Phosgen mit dem zur Konstanthaltung des pH-Wertes zugesetzten wäßrigen Alkali überstöchiometrische Mengen an Alkalichlorid gebildet. Außerdem bleiben bei pH-Werten über 10 die entstehenden Benzimidazolone in Form ihrer Alkalisalze vollständig oder teilweise in Lösung und müssen durch Zurückstellen des pH-Wertes mittels Säure wieder ausgefällt werden.

Zur Aufrechterhaltung eines konstanten pH-Wertes während der Reaktion des o-Phenylendiamins mit Phosgen dient Alkali- oder Erdalkalibase. Bevorzugt wird wäßriges Alkalihydroxid eingesetzt, welches in Form einer verdünnten, wäßrigen Lösung verwendet werden kann. Es kann auch die wäßrige Lösung eines Alkalisalzes einer schwachen mineralsäure oder organischen Säure, wie beispielsweise von Alkalicarbonat, Alkalihydrogencarbonat oder Alkaliacetat, eingesetzt werden. Grundsätzlich ist es auch möglich, den gewünschten pH-Wert mit geeigneten Pufferlösungen einzustellen, beispielsweise mit einer Phosphat- bzw. Acetatpufferlösung. Bevorzugt wird jedoch wäßrige Natriumhydroxidlösung eingesetzt, wobei die Konzentration zwischen 1% und 50 %, insbesondere zwischen 20 % und 50 % liegt. Diese wird während der Umsetzung des eingesetzten o-Phenylendiamins mit Phosgen in die Reaktionsmischung in dem Maße eindosiert, daß der gewünschte pH-Wert während der Reaktion konstant bleibt.

Überraschend ist beim erfindungsgemäßen Verfahren, daß praktisch kein Phosgen durch Wasser bzw. anwesende Base zersetzt wird, weshalb nur stöchiometrische Mengen an Phosgen und Lauge gemäß der allgemeinen Reaktionsgleichung

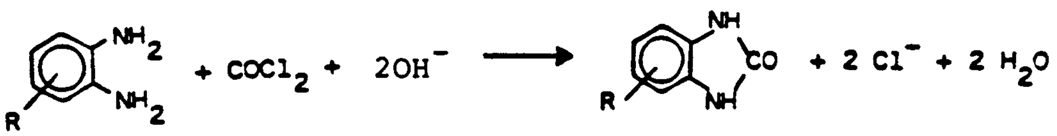

verbraucht werden.

Die beim erfindungsgemäßen Verfahren vollständig ausgefallenen Benzimidazolone können durch Filtration direkt isoliert und mit Wasser salzfrei gewaschen werden.

Nachstehend wird das erfindungsgemäße Verfahren näher erläutert:

o-Phenylendiamin bzw. substituierte o-Phenylendiamine werden in Wasser bei einer Temperatur zwischen 20 und 100 °C, vorzugsweise zwischen 70 und 100 °C, gelöst. Die Konzentration der o-Phenylendiamin-Lösung ist dabei von der Löslichkeit des jeweiligen Diamins bei der gewählten Temperatur abhängig. Im allgemeinen liegt die Konzentration der Lösungen im Bereich zwischen 5 und 30 Gewichtsprozent. Grundsätzlich können auch höhere Konzentrationen und damit Suspensionen der gegebenenfalls substituierten o-Phenylendiamine verwendet werden, was unter Umständen zu einem über die Stöchiometrie hinausgehenden Phosgenverbrauch führen kann. Die Ausbeuten und der Reingehalt der Produkte bleiben jedoch unverändert gut. Das Arbeiten in geringeren Konzentrationen ist im Prinzip ebenfalls möglich, jedoch aus ökonomischen Gründen nicht sinnvoll. Von entscheidender Bedeutung für das erfindungsgemäße Verfahren ist jedoch, wie schon ausgeführt, der pH-Wert während der Umsetzung der gegebenenfalls substituierten o-Phenylendiamine.

Das Molverhältnis o-Phenylendiamin zu Phosgen bewegt sich zwischen 1:1 und höchstens 1:1,5, vorzugsweise zwischen 1:1 und 1:1,2.

Das Molverhältnis von o-Phenylendiamin zu Alkali- oder Erdalkalibase beträgt 1:1 bis höchstens 1:3, vorzugsweise 1:2 bis 1:2,4.

Das erfindungsgemäße Verfahren kann sowohl bei Normaldruck als auch bei erhöhtem Druck durchgeführt werden.

Das erfindungsgemäße Verfahren stellt eine wesentliche Verbesserung gegenüber den zum Stand der Technik zählenden Verfahren dar, was sowohl für Ausbeuten (>90 %) und Reinheit (hohe Reinheit) der erhaltenen Benzimidazolone als auch für den ökologischen Aspekt gilt, da es mit dem erfindungsgemäßen Verfahren gelingt, den Phosgenverbrauch durch die gezielte pH-Führung annähernd stöchiometrisch zu halten und damit die zwangsläufig anfallende Menge an Alkalichlorid auf ein Minimum zu beschränken.

Die nachstehenden Beispiele dienen zur Erläuterung der Erfindung, ohne diese darauf zu beschränken.

**Beispiel 1** (Benzimidazolon-2)

Zu 108 g (1 mol) o-Phenylendiamin in 450 ml Wasser werden bei 80 °C über einen Zeitraum von 4 h 100 g (1,01 mol) Phosgen eingeleitet und durch gleichzeitiges Zudosieren von 407 g (2,04 mol) 20 %ige wäßrige Natronlauge der pH-Wert konstant bei 10 gehalten. Der Endpunkt der Reaktion wird durch Probe auf diazotierbares Amin festgestellt. Nach dem Abkühlen wird das ausgefallene Produkt abgesaugt, mit Wasser gewaschen und getrocknet. Man erhält auf diese Weise 131 g Benzimidazolon mit einem Schmelzpunkt von 308 °C, was einer Ausbeute von 98% der Theorie entspricht.

**Beispiel 2** (Benzimidazolon-2)

Zu 108 g (1 mol) o-Phenylendiamin in 800 ml Wasser werden bei 80 °C über einen Zeitraum von 4 h 100 g (1,01 mol) Phosgen eingeleitet und durch gleichzeitiges Zudosieren von 231 g (1,9 mol) 50 %ige wäßrige Natronlauge der pH-Wert konstant bei 6,5 gehalten. Nach dem Absaugen und Waschen mit Wasser erhält man 126 g Benzimidazolon-2, was einer Ausbeute von 94 % der Theorie entspricht.

**Beispiel 3** (5-Methyl-benzimidazolon-2)

Zu 61 g (0,5 mol) 4-Methyl-1,2-diaminobenzol in 450 ml Wasser werden bei 80 °C 53 g (0,54 mol) Phosgen eingeleitet und gleichzeitig mit 86 g (1,08 mol) 50 %ige wäßrige Natronlauge der pH-Wert bei 7,2 konstant gehalten. Nach dem Abkühlen erhält man 72 g ausgefallenes 5-Methyl-benzimidazolon-2 mit einem Schmelzpunkt von 290 °C, was einer Ausbeute von 97 der Theorie entspricht.

**Beispiel 4** (5-Chlorbenzimidazolon-2)

In eine Lösung von 142 g (1 mol) 4-Chlor-1,2-diaminobenzol in 1 l Wasser werden bei 80 °C nach Maßgabe des Beispiels 1 115g (1,16 mol) Phosgen und 459,2 g (2,3 mol) 20 %ige wäßrige Natronlauge bei einem pH-Wert von 8,0 eingeleitet bzw. zudosiert. Isoliert werden 161,5 g 5-Chlorbenzimidazolon mit einem Schmelzpunkt von 320 °C, was einer Ausbeute von 96 % der Theorie entspricht.

**Beispiele 5 - 10**

Wie in Beispiel 1 beschrieben, wurden zu 1 mol substituiertem o-Phenylendiamin in 1 l Wasser gleichzeitig Phosgen und Natronlauge eingeleitet bzw. eindosiert und der pH-Wert konstant bei 7,5 gehalten. Die Ergebnisse sind in der nachstehenden Tabelle zusammengefaßt.

| Substituiertes o-Phenylendiamin | Ausbeute an substituiertem Benzimidazolon | Schmelzpunkt |
|---|---|---|
| 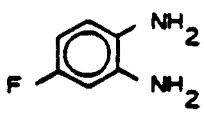 | 98,6 % | 300°C |
| 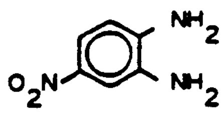 | 95,7 % | 298°C |
| 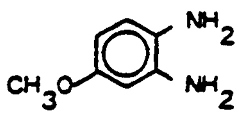 | 98,5 % | 256°C |
| 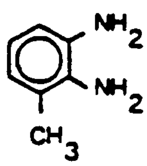 | 96,9 % | 300°C |

| Substituiertes o-Phenylendiamin | Ausbeute an substituiertem Benzimidazolon | Schmelzpunkt |
|---|---|---|
| 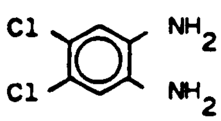 | 95,3 % | >340°C |
| 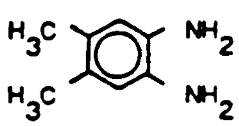 | 97,3 % | >340°C |

## Patentansprüche

1. Verfahren zur Herstellung von Benzimidazolonen der allgemeinen Formel (1)

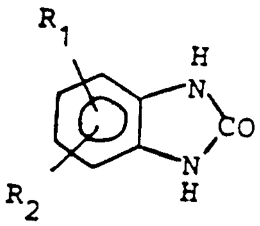

(1)

in welcher $R_1$ und $R_2$ gleich oder verschieden sind und Wasserstoff oder Halogenatome, Alkyl($C_1$-$C_4$)-, Alkoxy($C_1$-$C_4$)-, Trifluormethyl-, Phenyl-, Phenoxy- oder Nitrogruppen bedeuten, dadurch gekennzeichnet, daß man die Umsetzung von o-Phenylendiaminen der allgemeinen Formel (2)

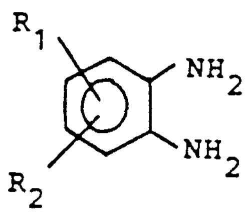

(2)

in welcher $R_1$ und $R_2$ die vorstehend genannten Bedeutungen haben, mit Phosgen in Wasser in Gegenwart einer Alkali- oder Erdalkalibase oder eines Salzes aus einem Alkali- oder Erdalkalihydroxid und einer schwachen anorganischen oder organischen Säure oder einer Mischung solcher Salze oder eines, geeigneten Puffersystems bei einem konstanten pH-Wert Zwischen 6 bis 12 und einer Temperatur von 20 bis 100 °C durchführt.

2. Verfahren nach Anspruch 1, dadurch gekennzeichnet, daß man die Umsetzung bei einem konstanten pH-Wert Zwischen 6,5 bis 11 durchführt.

3. Verfahren nach mindestens einem der Ansprüche 1 und 2, dadurch gekennzeichnet, daß man die Umsetzung bei einem konstanten pH-Wert Zwischen 8 bis 10 durchführt.

4. Verfahren nach mindestens einem der Ansprüche 1 bis 3, dadurch gekennzeichnet, daß man die Umsetzung in Gegenwart einer wäßrigen Natriumhydroxidlösung durchführt.

5. Verfahren nach mindestens einem der Ansprüche 1 bis 4, dadurch gekennzeichnet, daß man die Umsetzung bei einer Temperatur von 70 bis 100 °C durchführt.

## Claims

1. A process for the preparation of a benzimidazolone of the formula (1)

(1)

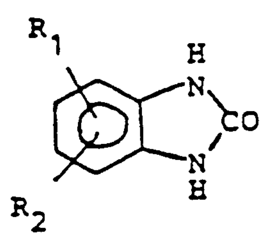

in which $R_1$ and $R_2$ are identical or different and denote hydrogen or halogen atoms, or alkyl ($C_1$-$C_4$),

alkoxy ($C_1$-$C_4$), trifluoromethyl, phenyl, phenoxy or nitro groups, which comprises reacting an o-phenylenediamine of the formula (2)

(2)

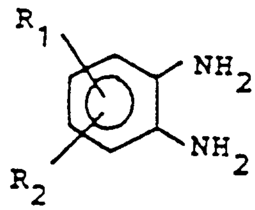

in which $R_1$ and $R_2$ have the abovementioned meanings, with phosgene in water in the presence of an alkali metal base or alkaline earth metal base or a salt of an alkali metal hydroxide or alkaline earth metal hydroxide and a weakly inorganic or organic acid or a mixture of such salts or a suitable buffer system, at a constant pH of between 6 and 12, and at a temperature of 20 to 100 °C.

**2.** The process as claimed in claim 1, wherein the reaction is carried out at a constant pH between 6.5 and 11.

**3.** The process as claimed in either of claims 1 and 2, wherein the reaction is carried out at a constant pH of between 8 and 10.

**4.** The process as claimed in any one of claims 1 to 3, wherein the reaction is carried out in the presence of an aqueous sodium hydroxide solution.

**5.** The process as claimed in any one of claims 1 to 4, wherein the reaction is carried out at a temperature of 70 to 100 °C.

**Revendications**

**1.** Procédé de préparation de benzimidazolones de formule générale (1)

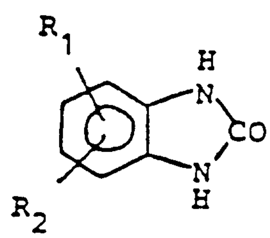

(1)

(dans laquelle $R_1$ et $R_2$ sont identiques ou différents et représentent chacun un atome d'hydrogène ou des atomes d'halogène(s), un groupe alkyle en $C_1$ à $C_4$ , alcoxy en $C_1$ à $C_4$, trifluorométhyle, phényle, phénoxy ou nitro), procédé caractérisé en ce qu'on effectue la réaction d'o-phénylène diamines de formule générale (2)

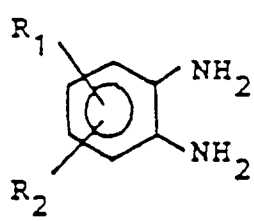

(2)

(dans laquelle $R_1$ et $R_2$ ont les sens précités) avec du phosgène dans de l'eau en présence d'une base alcaline ou alcalino terreuse ou d'un sel d'un hydroxyde alcalin ou alcalino terreux et d'un acide minéral

ou organique faible ou d'un mélange de tels sels ou d'un système tampon convenable à une valeur de pH constante comprise entre 6 et 12 et à une température de 20 à 100 °C.

**2.** Procédé selon la revendication 1, caractérisé en ce qu'on effectue la réaction à une valeur de pH constante comprise entre 6,5 et 11.

**3.** Procédé selon l'une au moins des revendications 1 et 2, caractérisé en ce qu'on effectue la réaction à une valeur de pH constante comprise entre 8 et 10.

**4.** Procédé selon l'une au moins des revendications 1 à 3, caractérisé en ce qu'on effectue la réaction en présence d'une solution aqueuse d'hydroxyde de sodium.

**5.** Procédé selon l'une au moins des revendications 1 à 4, caractérisé en ce qu'on effectue la réaction à une température de 70 à 100 °C.